# EUROPEAN PATENT SPECIFICATION

(11) **EP 1 960 019 B1**
(45) Date of publication and mention of the grant of the patent: **01.08.2012**
(21) Application number: 06817544.7
(22) Date of filing: 27.11.2006
(51) Int. Cl.: A61M 5/168, A61M 39/02, A61M 39/22, A61M 5/158, A61M 39/24

(54) **Flow control assembly including a valve and flow controller**
Durchflusskontrollanordnung mit einem Ventil und einem Durchflussregler
Ensemble de régulation d'écoulement comprenant une vanne et un régulateur d'écoulement

(30) Priority: 12.12.2005 AU 2005906976
(43) Date of publication of application: 27.08.2008
(73) Proprietor: Acu Rate Pty Limited, Mosman NSW 2088 (AU)
(72) Inventor: MILIJASEVIC, Zoran, Bayview, NSW 2104 (AU)
(74) Representative: Carter, Stephen John
(86) International application number: PCT/AU2006/001792
(87) International publication number: WO 2007/068030

(56) References cited:
- EP-A- 0 810 000
- EP-A- 1 129 740
- EP-B1- 0 518 972
- WO-A-03/041772
- GB-A- 1 261 246
- US-A- 4 245 636
- US-A- 4 439 182
- US-A- 4 714 462
- US-A- 4 743 235
- US-A- 5 098 405
- US-B1- 6 508 791

## Description

### Cross-Reference to Related Applications

The present application claims priority from Australian Provisional Patent Application No 2005906976 filed on 12 December 2005.

### Field of the Invention

This invention relates to a flow control assembly for an intravenous (IV) administration set.

### Background to the Invention

IV administration sets are commonly used for infusing fluids into a patient's body. The flow rate of fluid through the administration set from a reservoir of the fluid is governed by a pressure head. The pressure head is generated by having the reservoir arranged at an elevated location relative to the entry point of fluid into the patient's body and/or by use of a pressure generating device such as a pump.

It is necessary to maintain the infusion rate of the fluid at a predetermined rate to ensure that the patient receives the correct dosage of the fluid being dispensed.

There are three main factors controlling the infusion rate of the fluid. These are, firstly, the pressure head created, secondly, the friction or resistance to flow in the system and, thirdly, the viscosity or density of the fluid being infused.

The pressure head obtained by an elevated reservoir may change due to the back pressure. The back pressure created can cause changes in the flow rate of the fluid and the pressure may change inadvertently, for example, by a patient turning over in bed or by standing up. Viscosity of the fluid may change due to ambient factors such as changes in the ambient temperature, etc.

It has been proposed to use an orifice plate to obtain a constant flow rate of the fluid to be infused independent of the viscosity of the fluid being infused. However, a needle assembly of an IV administration set is also sometimes used for injecting fluids into a patient. An orifice plate downstream of the injection point may impede injection of the fluid due to the small dimensions of the orifice. Also, there is the danger of back flow of bodily fluids from the patient's body when the administration set is removed. This bodily fluid may come into contact with the person removing the set and could have adverse consequences due to blood borne diseases and the like.

EP 0810000 discloses a flow rate control device which does not constitute an obstruction when it is connected to a liquid medicine continuous injector, which is not influenced by the body temperature of a patient and which can perform a priming operation of the liquid medicine continuous injector in a short period of time. The control device is provided with a reservoir, a flow rate control tube and release valve which are preferably incorporated in a housing. The housing is provided with a through hole such that the reservoir can be pressed by a pressing means, and is also provided with a liquid medicine inlet and a liquid medicine outlet for the reservoir. A check valve is preferably provided in the liquid medicine inlet and the flow rate control tube and release valve are provided in the liquid medicine outlet

EP 0518972 discloses an in-line fluid flow controller for use with a fluid administration set for infusion of fluid into the body of a patient. The administration set comprises a reservoir of fluid connected to a supply line having a discharge orifice. The fluid flow controller comprises a housing having a chamber therein, an inlet to and an outlet from the chamber, the housing being adapted to receive therewithin at least one flow restrictor each having an orifice or orifices configured to control the rate of fluid flow therethrough and into the body of a patient such that the fluid is infused into a patient at a predetermined constant flow rate or at a flow rate within predetermined limits via the discharge orifice.

WO 03/041772 discloses a device for preventing reverse flow of a medical solution during an infusion. The device is included in a solution injecting set consisting of a solution dropper connected to a reservoir for controlling the supply of solution from the reservoir. A hose extends from the dropper and a solution controller is mounted to the hose. A needle is mounted to the end of the hose. The device consists of an anti-reverse chamber formed at the hose with a perforated partition wall dividing the chamber into two parts having a guide hole at the centre. A guide rod supporting a check plate is inserted in the guide hole such that the guide rod can be moved in a direction perpendicular to the partition wall.

EP 1129740 and GB 1262146 disclose further prior art flow control assemblies including multiple check valves.

### Summary of the Invention

According to the invention, there is provided a flow control assembly, the flow control assembly including
a housing having an upstream end which communicates with a source of fluid to be dispensed, in use, and a downstream end which communicates with a supply conduit connectable to the downstream end with a passage being defined through the housing between the upstream end and the downstream end of the housing; and
a valve arrangement, characterised in that the valve arrangement comprises at least two valves arranged in series in the passage of the housing with a first valve of the series being an upstream valve and a second valve of the series being a downstream valve, each valve having an operating member which opens at a predetermined fluid pressure with the downstream valve opening at a substantially lower pressure than the upstream valve, the valves controlling the flow of fluid through the passage and in that a flow controller in the form of an orifice plate is mounted in an operating member of the first valve of the valve arrangement, the orifice plate facilitating flow of fluid through the passage regardless of whether or not the first valve of the valve arrangement is open or closed.

In this specification, the term "state of the valve arrangement" means, unless the context clearly indicates otherwise, whether or not the valve arrangement is open or closed.

The housing may be connectable to, or form part, of an outlet of a reservoir of the fluid. The reservoir may function as the source of fluid. Instead, the housing may be mounted in-line in the supply conduit or form part of the supply conduit. Further, the upstream end of the housing may be shaped to receive an end of a syringe. Thus, the upstream end of the housing may be in the form of a female component of a Luer lock.

The operating member of at least the first valve may comprise a pair of opposed leaves which are arranged in a normally closed configuration to form a duck bill valve.

It will be appreciated that the orifice plate serves to control the flow rate of the fluid through the passage in a manner substantially independently of the viscosity of the fluid.

The operating member of the second valve may be configured to open at a pressure caused only by the passage of fluid through the orifice plate. Further, opening of the second valve is pressure dependent, the second valve being configured to open at fluid pressures corresponding to an infusion flow rate of fluid through the orifice plate.

Opening of the first valve of the valve arrangement may be flow rate dependent, the first valve being configured to open in response to a fluid being dispensed through the housing from a syringe connected to the upstream end of the housing.

The second valve of the valve arrangement may inhibit back flow of fluid in the conduit and therefore reduce the risk of users of a system incorporating the flow control assembly coming into contact with bodily fluids of a patient.

The invention extends also to an intravenous administration set which includes a flow control assembly as described above.

### Brief Description of the Drawings

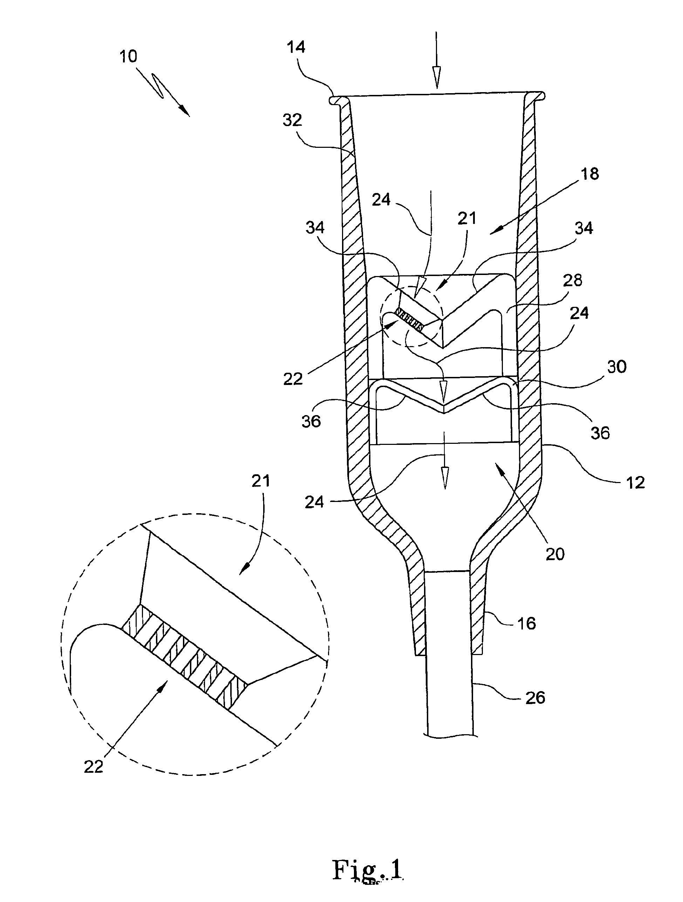

Fig.1 shows a schematic, sectional side view of a flow control assembly in accordance with an embodiment of the invention.

### Detailed Description of Exemplary Embodiment

In Fig. 1, reference numeral 10 generally designates a flow control assembly in accordance with an embodiment of the invention. While the assembly 10 may have uses in other applications, it will be described below with reference to its application in an intravenous (IV) administration set.

The flow control assembly comprises an elongate housing 12 having an upstream end 14 and a downstream end 16. A passage 18 is defined through the housing 12.

A valve arrangement 20 is arranged in the passage 18 of the housing 12 intermediate the upstream end 14 and the downstream end 16 of the housing 12.

A flow controller 21, comprising an orifice plate 22, is associated with the valve arrangement 20. The flow controller 21 facilitates flow of a fluid, or liquid, such as a saline solution, through the passage 18 regardless of a state of the valve arrangement 20.

The upstream end 14 is connectable to a source of liquid (not shown). Generally, the assembly 10 is used with an IV administration set and the source of liquid is a reservoir of the liquid which is to be infused at a predetermined flow rate into a patient's body. This is achieved by the liquid flowing through the orifice plate 22 as indicated by arrows 24.

The downstream end 16 of the housing 12 mounts a supply conduit 26. The supply conduit 26 leads to a needle assembly (not shown), a needle of which is inserted into a vein of the patient's body for the infusion of the liquid.

The valve arrangement 20 comprises a first valve 28 and a second valve 30 arranged downstream of the first valve 28.

The first valve 28 is configured to open at a predetermined pressure of liquid upstream of the valve 28. The valve 28 is designed to open when liquid is to be injected into the patient from a source other than the reservoir of the IV administration set, for example, when liquid is to be injected from a syringe.

For this purpose, the upstream end 14 of the housing 12 defines a female Luer lock formation 32 into which a male Luer lock of the syringe is receivable for injecting the liquid into the patient.

The valve 28 has a pair of operating members 34 arranged in a duck-bill formation. The orifice plate 22 is arranged in one of the operating members 34 so that liquid at the required infusion flow rate can pass through the housing 12 regardless of the state of the valve 28, i.e. regardless of whether the valve 28 is in an open configuration or a closed configuration.

The second valve 30 has a pair operating members 36 also arranged in a duck-bill configuration, which are configured to open at an extremely low pressure and a pressure significantly lower than the pressure at which the operating members 34 of the valve 28 open. More specifically, the operating members 36 of the valve 30 are configured to open at fluid pressures corresponding to the infusion flow rate of the liquid through the orifice plate 22. Further, the operating members 36 of the valve 30 can be tailored to open at different infusion flow rates.

The purpose of the valve 30 is to inhibit backflow of fluids, such as blood, when the administration set is removed from the assembly 10.

In use, the upstream end 14 of the housing 12 is connected to an outlet of the reservoir of liquid to be infused via an administration set. After suitable priming, liquid passes through the orifice plate 22 of the flow controller 21 and causes the operating member 36 of the valve 30 to open so that liquid passes into the conduit 26 and, via the needle assembly, into the patient's body at the required flow rate.

When it is required to inject a substance into the patient, the housing 12 is removed from the outlet of the reservoir and the male Luer lock of the syringe is inserted into the upstream end 14 of the housing 12. Expulsion of the liquid from the syringe causes the valve 28 to open and, as a result, also the valve 30. The liquid can thus be injected at a much higher rate than the flow rate through the flow controller 21.

After completion of the injection of the liquid, the housing 12 is reattached to the reservoir to allow continued infusion of the liquid from the reservoir.

When the needle assembly is to be removed from the patient's body, the supply of liquid from the reservoir is, firstly, stopped. This may create a low pressure region upstream of the valve 30. The valve 30 remains closed retaining pressure in the supply conduit 26. Thus, when the male Luer assembly of the IV administration set is removed from the assembly 10, the back flow of bodily fluids from the patient is inhibited.

It is a first advantage of the invention that a flow control assembly 10 is provided which facilitates the use of the same device, both for infusion and for injection of liquids. Secondly, the flow control assembly 10 inhibits the back flow of bodily fluids from a patient's body thereby inhibiting the chances of contamination of a user by such bodily fluids.

## Claims

1. A flow control assembly (10) for an intravenous administration set, the flow control assembly (10) including
a housing (12) having an upstream end (14) which communicates with a source of fluid to be dispensed, in use, and a downstream end (16) which communicates with a supply conduit (26) connectable to the downstream end (16) with a passage (18) being defined through the housing (12) between the upstream end (14) and the downstream end (16) of the housing (12); and
a valve arrangement (20) **characterised in that** the valve arrangement (20) comprises at least two valves (28, 30) arranged in series in the passage (18) of the housing (12) with a first valve (28) of the series being an upstream valve and a second valve (30) of the series being a downstream valve, each valve (28, 30) having an operating member (34, 36) which opens at a predetermined fluid pressure with the downstream valve (30) opening at a substantially lower pressure than the upstream valve (28), the valves (28, 30) controlling the flow of fluid through the passage (18) and **in that** a flow controller (21) in the form of an orifice plate (22) is mounted in an operating member (34) of the first valve (28) of the valve arrangement (20), the orifice plate (22) facilitating flow of fluid through the passage (18) regardless of whether or not the first valve (28) of the valve arrangement (20) is open or closed.

2. The assembly (10) of claim 1 in which the operating member (34) of at least the first valve (28) comprises a pair of opposed leaves (34) which are arranged in a normally closed configuration to form a duck bill valve.

3. The assembly (10) of claim 1 or claim 2 in which opening of the second valve (30) is pressure dependent, the second valve (30) being configured to open at fluid pressures corresponding to an infusion flow rate of fluid through the orifice plate (22).

4. The assembly (10) of any one of the preceding claims in which opening of the first valve (28) of the valve arrangement (20) is flow rate dependent, the first valve (28) being configured to open in response to a fluid being dispensed through the housing (12) from a syringe connected to the upstream end (14) of the housing (12).

5. An intravenous administration set which includes a flow control assembly as claimed in any one of the preceding claims.

## Patentansprüche

1. Durchflussregelungsanordnung (10) für ein intravenöses Verabreichungsset, wobei die Durchflussregelungsanordnung (10) Folgendes umfasst:
ein Gehäuse (12) mit einem stromaufwärtigen Ende (14), das während der Verwendung mit einer Quelle des abzugebenden Fluids kommuniziert, und einem stromabwärtigen Ende (16), das mit einer mit dem stromabwärtigen Ende (16) verbindbaren Versorgungsleitung (26) kommuniziert, wobei zwischen dem stromaufwärtigen Ende (14) und dem stromabwärtigen Ende (16) des Gehäuses (12) ein Durchlass (18) durch das Gehäuse (12) definiert ist; und
eine Ventilanordnung (20), die **dadurch gekennzeichnet ist, dass** die Ventilanordnung (20) zumindest zwei Ventile (28, 30) umfasst, die im Durchlass (18) des Gehäuses (12) in Serie geschaltet sind, wobei ein erstes Ventil (28) der Serienschaltung ein stromaufwärtiges Ventil ist und ein zweites Ventil (30) der Serienschaltung ein stromabwärtiges Ventil ist, wobei jedes Ventil (28, 30) ein Betriebselement (34, 36) aufweist, das bei einem vorbestimmten Fluiddruck öffnet, wobei das stromabwärtige Ventil (30) sich bei einem deutlich niedrigeren Druck öffnet als das stromaufwärtige Ventil (28), wobei die Ventile (28, 30) den Fluiddurchfluss durch den Durchlass (18) steuern, und dass ein Durchflussregler (21) in Form einer Blendenplatte (22) in einem Betriebselement (34) des ersten Ventils (28) der Ventilanordnung (20) angebracht ist, wobei die Blendenplatte (22) den Durchfluss von Fluid durch den Durchlass (18) erleichtert, unabhängig davon, ob das erste Ventil (28) der Ventilanordnung (20) geöffnet oder geschlossen ist.

2. Anordnung (10) nach Anspruch 1, worin das Betriebselement (34) zumindest des ersten Ventils (28) ein Paar gegenüberliegender Flügel (34) umfasst, die in einer normalerweise geschlossenen Konfiguration angeordnet sind, um ein Lippenventil zu bilden.

3. Anordnung (10) nach Anspruch 1 oder Anspruch 2, worin das Öffnen des zweiten Ventils (30) druckabhängig ist, wobei das zweite Ventil (30) so konfiguriert ist, dass es sich bei einem Fluiddruck öffnet, welcher der Infusionsdurchflussgeschwindigkeit von Fluid durch die Blendenplatte (22) entspricht.

4. Anordnung (10) nach einem der vorangegangenen Ansprüche, wobei das Öffnen des ersten Ventils (28) der Ventilanordnung (20) durchflussgeschwindigkeitsabhängig ist, wobei das erste Ventil (28) so konfiguriert ist, dass es sich als Reaktion auf die Abgabe eines Fluids von einer Spritze durch das Gehäuse (12) öffnet, die am stromaufwärtigen Ende (14) des Gehäuses (12) angeschlossen ist.

5. Intravenöses Verabreichungsset, das eine Durchflussregelungsanordnung nach einem der vorangegangenen Ansprüche umfasst.

## Revendications

1. Ensemble de régulation d'écoulement (10) pour un ensemble d'administration intraveineuse, l'ensemble de régulation d'écoulement (10) comprenant
un boîtier (12) présentant une extrémité amont (14) qui communique avec une source de fluide à distribuer, en cours d'utilisation, et une extrémité aval (16) qui communique avec un conduit d'amenée (26) pouvant être relié à l'extrémité aval (16) avec un passage (18) défini à travers le boîtier (12) entre l'extrémité amont (14) et l'extrémité aval (16) du boîtier (12); et
un agencement de vannes (20), **caractérisé en ce que** l'agencement de vannes (20) comprend au moins deux vannes (28, 30) agencées en série dans le passage (18) du boîtier (12), une première vanne (28) de la série étant une vanne amont, et une deuxième vanne (30) de la série étant une vanne aval, chaque vanne (28, 30) ayant un élément fonctionnel (34, 36) qui s'ouvre à une pression de fluide prédéterminée, la vanne aval (30) s'ouvrant à une pression sensiblement plus basse que la vanne amont (28), les vannes (28, 30) réglant l'écoulement de fluide à travers le passage (18), et **en ce qu'**un dispositif de commande d'écoulement (21) sous la forme d'une plaque à orifice (22) est installé dans un élément fonctionnel (34) de la première vanne (28) de l'agencement de vannes (20), la plaque à orifice (22) facilitant l'écoulement du fluide à travers le passage (18) indépendamment de ce que la première vanne (28) de l'agencement de vannes (20) soit ouverte ou fermée.

2. Ensemble (10) selon la revendication 1, dans lequel l'élément fonctionnel (34) d'au moins la première vanne (28) comprend deux feuilles opposées (34) qui sont agencées en une configuration normalement fermée pour former une vanne à bec de canard.

3. Ensemble (10) selon la revendication 1 ou la revendication 2, dans lequel l'ouverture de la deuxième vanne (30) dépend de la pression, la deuxième vanne (30) étant configurée pour s'ouvrir à des pressions de fluide correspondant à un débit d'écoulement d'infusion du fluide à travers la plaque à orifice (22).

4. Ensemble (10) selon l'une quelconque des revendications précédentes, dans lequel l'ouverture de la première vanne (28) de l'agencement de vannes (20) dépend du débit d'écoulement, la première vanne (28) étant configurée pour s'ouvrir en réponse à la distribution d'un fluide à travers le boîtier (12) par une seringue reliée à l'extrémité amont (14) du boîtier (12).

5. Ensemble d'administration intraveineuse, qui comprend un ensemble de régulation d'écoulement tel que revendiqué dans l'une quelconque des revendications précédentes.
